# FASCICULE DE BREVET EUROPEEN

(11) **EP 3 316 934 B1**
(45) Date de publication et mention de la délivrance du brevet: **17.04.2019**
(21) Numéro de dépôt: 16742363.1
(22) Date de dépôt: 30.06.2016
(51) Int. Cl.: A61M 5/20, A61M 5/30

(54) **DISPOSITIF D'INJECTION SANS AIGUILLE À BOUCHON DE FERMETURE POSITIONNABLE ANGULAIREMENT**
NADELLOSER INJEKTOR MIT STOPPER, DER IN EINEM WINKEL POSITIONIERT WERDEN KANN
NEEDLELESS INJECTOR WITH STOPPER POSITIONABLE AT AN ANGLE

(30) Priorité: 30.06.2015 FR 1556157
(43) Date de publication de la demande: 09.05.2018
(73) Titulaire: Crossject, 21000 Dijon (FR)
(72) Inventeur: VIVIEN, Gilles, 92240 Malakof (FR); VIGOT, Xavier, 21260 Veronnes (FR)
(74) Mandataire: Chevalier, Renaud Philippe
(86) Numéro de dépôt international: PCT/FR2016/051659
(87) Numéro de publication internationale: WO 2017/001798

(56) Documents cités:
- FR-A1- 2 815 544
- FR-A1- 2 978 021
- GB-A- 2 046 228

## Description

Le domaine technique de l'invention est celui des dispositifs d'injection sans aiguille, préremplis et jetables, fonctionnant avec une source d'énergie comme par exemple un générateur de gaz, et utilisés pour les injections intradermiques, souscutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans le corps du dispositif et qui est obturé par un bouchon-piston amont et un bouchon-piston aval entre lesquels est contenu le principe actif liquide.

L'extrémité libre aval du réservoir coopère avec une buse d'injection qui délimite au moins un canal d'injection s'étendant axialement suivant un axe d'injection.

Le générateur de gaz est conçu pour générer un gaz sous pression qui entraîne en déplacement les bouchon-pistons pour injecter le principe actif à travers la peau du patient en passant par la buse d'injection.

De plus, le dispositif d'injection comporte un capot creux qui enveloppe le corps et qui délimite une ouverture inférieure adaptée pour le passage de la buse d'injection.

L'extrémité libre de la buse d'injection qui fait saillie hors du corps et du capot est protégée par un opercule amovible et un bouchon qui loge l'opercule.

A cet effet, le bouchon comporte une portion globalement tubulaire qui délimite un logement adapté pour loger l'opercule et la partie aval inférieure saillante de la buse.

L'opercule est par exemple fixé sur la buse de façon amovible par un moyen de verrouillage du type à baïonnette.

Le déverrouillage de l'opercule, qui est solidaire en pivotement du bouchon, est effectué par pivotement du bouchon par rapport au capot, autour de l'axe d'injection, entre une position de fermeture dans laquelle le bouchon et le capot sont alignés en ne formant aucune aspérité, et une position d'ouverture dans laquelle l'ensemble formé par le bouchon et l'opercule peuvent être retirés.

A cet effet, l'opercule comporte une bague annulaire qui est montée sur sa périphérie et qui délimite une paroi périphérique crantée dont les crans coopèrent avec des dents du bouchon.

Une telle conception par coopération de crans et de dents limite le nombre de positions angulaires, autour de l'axe de pivotement, entre le bouchon et l'ensemble formé par la bague et l'opercule.

Par conséquent, lorsque le bouchon occupe sa position de fermeture, un décalage angulaire peut être observé entre le bouchon et le capot, ce décalage engendrant un désalignement du bouchon et du capot assimilé à un défaut visuel du dispositif d'injection.

De plus, la bague crantée étant du type à crémaillère, elle n'autorise qu'un sens d'ouverture du bouchon, par exemple selon un sens trigonométrique, ce qui rend le dispositif d'injection peu adapté à une partie des utilisateurs.

La présente invention vise notamment à résoudre ces inconvénients et se rapporte pour ce faire à un dispositif d'injection sans aiguille comportant :
- un corps,
- un générateur de gaz,
- un système d'injection qui s'étend axialement suivant un axe d'injection et qui comprend au moins, d'amont en aval suivant le sens d'injection, un piston, un réservoir de principe actif, une buse d'injection qui délimite au moins un canal d'injection,
- un capot qui loge le corps du dispositif,
- un opercule amovible adapté pour protéger une extrémité aval inférieure de la buse d'injection,
- un bouchon qui loge l'opercule et qui est monté pivotant autour de l'axe d'injection entre une position de fermeture dans laquelle le bouchon est verrouillé sur le dispositif d'injection et une position d'ouverture dans laquelle le bouchon est déverrouillé du dispositif d'injection, caractérisé en ce que le dispositif comporte un élément de blocage qui est interposé radialement entre l'opercule et le bouchon, ledit élément de blocage étant conçu pour bloquer par friction le pivotement relatif de l'opercule et du bouchon, autour de l'axe d'injection, au cours de l'entraînement en pivotement du bouchon.

Ainsi, l'élément de blocage offre la possibilité de positionner l'opercule par rapport au bouchon de manière précise, et offre également, par conséquent, un positionnement angulaire précis du bouchon par rapport au capot, de sorte que le bouchon, dans sa position de fermeture, est parfaitement aligné avec le capot du dispositif.

Selon un exemple de réalisation préféré, l'élément de blocage est une bague qui est fixée sur une paroi périphérique cylindrique de l'opercule autour de l'axe d'injection.

De préférence, l'élément de blocage est réalisé en matériau déformable élastiquement.

Plus particulièrement, l'élément de blocage est réalisé en élastomère.

Aussi, l'élément de blocage est réalisé en surmoulage sur l'opercule.

Selon une autre caractéristique, le bouchon comporte une portion globalement tubulaire qui délimite un logement adapté pour loger l'opercule et l'extrémité aval de la buse, ladite portion tubulaire étant fermée à une extrémité aval par un disque de fond amovible, ledit disque comportant une pluralité de pattes de serrage qui s'étendent axialement depuis une face supérieure du disque et qui sont conçues pour être chacune en appui radial sur l'élément de blocage pour pincer et bloquer l'élément de blocage en pivotement.

Cette caractéristique permet un montage précis de l'opercule sur le bouchon, suivant une infinité de positions angulaires.

Pour favoriser le pincement de l'opercule, chaque patte de serrage est déformable élastiquement au moins radialement vers le centre de l'opercule.

De même, chaque patte de serrage présente une nervure axiale, formant dent, qui est adaptée pour pénétrer radialement sensiblement dans l'élément de blocage pour bloquer ledit élément de blocage en pivotement par rapport au bouchon.

De plus, chaque patte de serrage est en appui radial contre une paroi interne du bouchon pour contraindre lesdites pattes radialement vers l'élément de blocage.

Pour éviter l'intrusion d'impuretés dans les canaux d'injection, l'opercule présente une collerette globalement annulaire qui s'étend autour de l'axe d'injection et qui est adapté pour coopérer avec l'extrémité aval de la buse.

De plus, le dispositif d'injection comporte un premier moyen de verrouillage du type à baïonnette adapté pour verrouiller le bouchon sur le corps du dispositif, au cours de l'entraînement du bouchon entre sa position d'ouverture et sa position de fermeture.

Aussi, le dispositif d'injection comporte un second moyen de verrouillage du type à baïonnette adapté pour verrouiller l'opercule sur la buse au cours de l'entraînement du bouchon entre sa position d'ouverture et sa position de fermeture.

Selon un exemple de réalisation, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue en perspective éclatée axialement, qui illustre un dispositif d'injection sans aiguille comportant un opercule et un bouchon de fermeture positionnable angulairement, selon l'invention ;
- la figure 2 est une vue de détail en perspective de la figure 1, qui illustre la buse, l'opercule et le bouchon du dispositif de la figure 1 ;
- la figure 3 est une vue en coupe axiale transversale qui illustre l'opercule de la figure 1 agencée sur la buse et le bouchon en position de fermeture ;
- la figure 4 est une vue de détail en coupe axiale longitudinale qui illustre l'opercule de la figure 1 agencée sur la buse et le bouchon en position de fermeture ;
- la figure 5 est une vue de détail en perspective en coupe radiale qui illustre l'opercule et sa bague périphérique coopérant avec les pattes du disque du bouchon ;
- la figure 6 est une vue de détail en perspective en coupe radiale qui illustre l'opercule et sa bague périphérique coopérant avec les pattes du disque du bouchon.

Dans la description et les revendications, pour clarifier la description et les revendications, on adoptera à titre non limitatif la terminologie longitudinal, vertical et transversal en référence au trièdre L, V, T indiqué aux figures.

A noter que dans la présente demande de brevet, les termes « amont » et «aval » doivent s'entendre par rapport à la circulation du principe actif à l'intérieur du dispositif d'injection, suivant un sens d'injection.

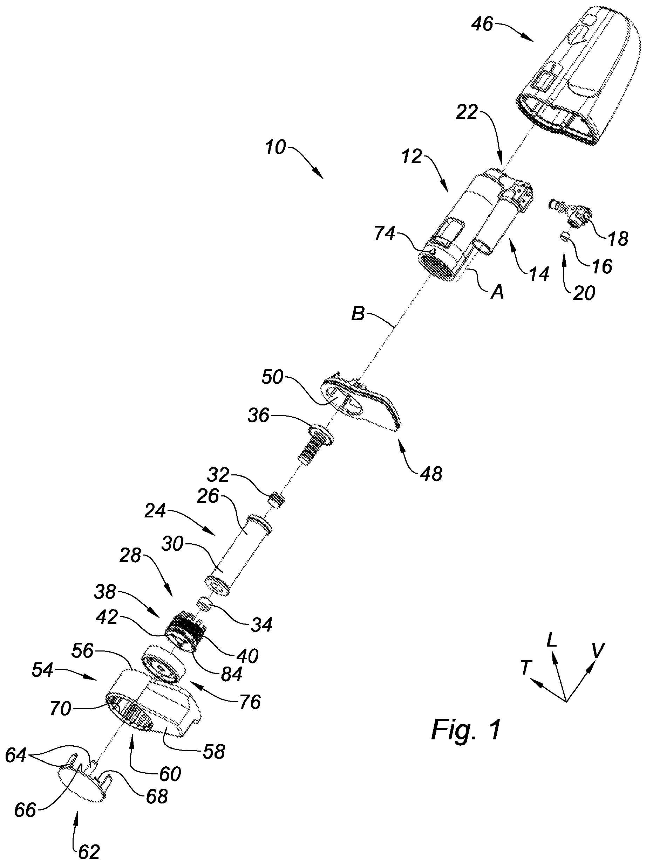

De plus, dans la présente demande, les termes « haut », « bas », « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures 1 à 6, sans référence à la gravité terrestre.

On a représenté à la figure 1 un dispositif 10 d'injection sans aiguille, ou seringue sans aiguille, qui comporte un corps 12 en forme de U comprenant successivement un dispositif de percussion 14, une amorce 16, une charge pyrotechnique 18, ces trois éléments constituant un générateur de gaz 20, une chambre d'expansion 22, un réservoir 24 contenant le principe actif liquide 26 et un système d'injection 28.

Le générateur de gaz 20 constitue un premier sous-ensemble linéaire du corps 12 qui s'étend axialement suivant un premier axe A vertical, et le réservoir 24 contenant le principe actif 26 et le système d'injection 28 forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un bouchon-piston amont 32 et un bouchon-piston aval 34 entre lesquels est contenu le principe actif liquide 26, les bouchons-pistons étant réalisés en matériau déformable élastiquement à base d'élastomère.

Le réservoir 24 est inséré dans le corps 12 et est bloqué verticalement, d'une part, à sa partie amont par une pièce cylindrique 36 munie d'une ouverture centrale permettant de mettre en communication le bouchon-piston amont 32 avec la chambre d'expansion 22, et, d'autre part, à sa partie aval par une buse 38 d'injection.

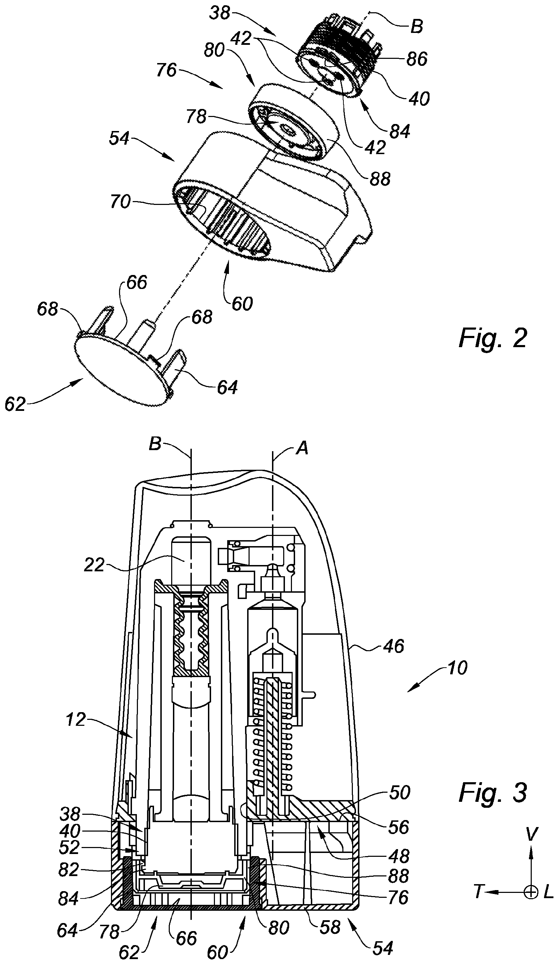

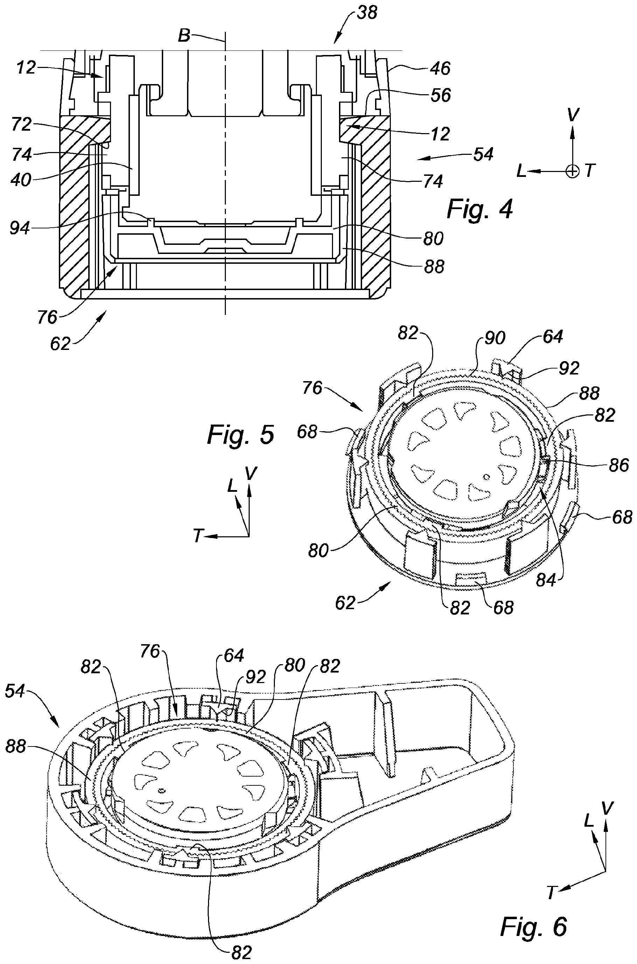

La buse 38, visible plus en détail aux figures 2 et 4, présente une forme cylindrique suivant l'axe B d'injection qui est délmitée par une face cylindrique périphérique 40 munie d'un filetage, le filetage étant prévu pour coopérer avec un taraudage complémentaire formé sur la paroi interne de l'extrémité aval du corps 12.

De plus, la buse 38 délimite trois canaux 42 axiaux d'injection parallèles à l'axe B d'injection, illustrés à la figure 2.

Selon la figure 3, le corps 12 est enveloppé par un capot 46 creux qui délimite une ouverture inférieure fermée par une semelle 48 horizontale formant fond de capot.

La semelle 48 délimite un passage circulaire 50 autour de l'axe B d'injection qui est adapté pour le passage de la buse 38 d'injection et de l'extrémité aval du corps 12, de sorte que la buse 38 comporte un tronçon inférieur 52 faisant saillie verticalement vers le bas hors du capot 46.

Aussi, le dispositif 10 d'injection est équipé d'un bouchon 54 qui est délimité verticalement par une face supérieure 56 ouverte en appui sur la semelle 48 du capot 46, et une face inférieure 58 fermée globalement plane.

Le bouchon 54 délimite un logement globalement tubulaire qui s'étend axialement suivant l'axe B d'injection et qui débouche dans la face inférieure 58 du bouchon 54 en formant un passage 60 circulaire fermé par un disque 62 de fond amovible.

Le disque 62 de fond comporte six pattes 64 de serrage qui s'étendent axialement parallèlement à l'axe B d'injection, depuis une face supérieure 66 du disque 62.

De plus, pour permettre la fixation du disque 62 sur le bouchon 54, le disque 62 comporte quatre dents 68 déformables élastiquement adaptées chacune pour coopérer avec une gorge 70 annulaire interne formée par le bouchon 54.

Selon un autre aspect, le bouchon 54 est monté pivotant autour de l'axe B d'injection entre une position de fermeture dans laquelle le bouchon 54 est positionné dans le prolongement du capot 46, de sorte que le bouchon 54 et le capot 46 forment une coquille homogène sans aspérité, et une position d'ouverture dans laquelle le bouchon 54 est pivoté angulairement d'environ 60 degrés autour de l'axe B d'injection.

Dans sa position de fermeture, le bouchon 54 est verrouillé sur le reste du dispositif 10 d'injection. A l'inverse, dans sa position d'ouverture, le bouchon 54 est apte à être retiré du reste du dispositif 10 pour permettre l'accès à la buse 38 d'injection et pour réaliser une injection.

Dans ce but, le dispositif 10 d'injection comporte un premier moyen de verrouillage du type à baïonnette adapté pour verrouiller le bouchon 54 sur le corps 12 du dispositif 10, au cours de l'entraînement du bouchon 54 entre sa position d'ouverture et sa position de fermeture.

A cet effet, comme on peut le voir à la figure 4, la face supérieure 56 du bouchon 54 délimite une gorge 72 qui est conçue pour coopérer avec deux ergots 74 (dont un est représenté à la figure 1) qui font saillie radialement depuis une extrémité inférieure du corps 12.

Conformément à l'invention, le dispositif 10 d'injection comporte un opercule 76 amovible adapté pour obturer l'extrémité aval inférieure de la buse 38 d'injection.

Comme on peut le voir aux figures 2 et 3, l'opercule 76 présente la forme d'une coupelle, qui présente une paroi de fond 78 radiale et une couronne 80 périphérique cylindrique s'étendant autour de l'axe B d'injection.

L'opercule 76 est verrouillé sur l'extrémité libre de la buse 38 d'injection de façon amovible par un second moyen de verrouillage du type à baïonnette.

A cet effet, en référence à la figure 5, l'opercule 76 comporte trois ergots 82 qui font saillie radialement vers le centre de l'opercule 76, depuis une paroi interne de la couronne 80 de l'opercule 76, et qui sont répartis angulairement de façon régulière à 120 degrés autour de l'axe B d'injection.

Complémentairement, l'extrémité libre aval de la buse 38 forme une collerette 84 de verrouillage qui s'étend radialement à la périphérie de la buse 38 et qui présente trois échancrures 86 adaptées pour permettre le passage axial des ergots 82 de l'opercule 76 au-delà de la colerette 84.

Selon un autre aspect de l'invention, le dispositif 10 d'injection comporte une bague 88 globalement cylindrique, formant élément de blocage, qui s'étend autour de l'axe B d'injection et qui est fixée sur la face externe de la couronne 80 périphérique de l'opercule 76.

En référence à la figure 5, la face externe de la couronne 80 périphérique de l'opercule 76 forme des stries 90 axiales qui bloquent la bague 88 en rotation sur l'opercule 76.

En outre, la bague 88 est agencée pour être interposée radialement entre la couronne 80 périphérique de l'opercule 76 et les pattes 64 du disque 62 du bouchon 54.

Aussi, la bague 88 est conçue pour bloquer le pivotement relatif de l'opercule 76 et du bouchon 54 par friction, autour de l'axe B d'injection, par l'intermédiaire des pattes 64 du disque 62 de fond du bouchon 54.

On entend par friction une interaction par frottement qui s'oppose au mouvement relatif entre l'opercule 76 et l'ensemble formé par le bouchon 54 et le disque 62, en opposition à un couplage mécanique du type à cran ou à crémaillère.

Une telle conception permet de positionner angulairement l'opercule 76 par rapport au bouchon 54, autour de l'axe B d'injection, suivant une infinité de positions angulaires.

Pour permettre le blocage en pivotement par friction, la bague 88 de blocage est réalisée en matériau déformable élastiquement.

De préférence, la bague 88 de blocage est réalisée en élastomère, et plus particulièrement en élastomère thermoplastique.

Avantageusement, chaque patte 64 de serrage appartenant au disque 62 est déformable élastiquement radialement vers l'axe B d'injection.

De même, chaque patte 64 de serrage présente une nervure axiale 92 saillante radialement, formant dent, qui est adaptée pour pénétrer radialement sensiblement dans la bague 88 de blocage pour pincer et bloquer la bague 88 en pivotement par rapport au bouchon 54, autour de l'axe B d'injection.

De plus, chaque patte 64 de serrage est en appui radial contre une paroi interne du bouchon 54 pour empêcher les pattes 64 de s'écarter radialement vers l'extérieur du bouchon 54 et pour contraindre les pattes 64 en appui radial sur la bague 88.

Selon un exemple de réalisation préféré, la bague 88 de blocage est réalisée en surmoulage sur l'opercule 76 qui est réalisée au moyen d'un matériau du type thermoplastique plus rigide.

Selon un autre aspect, l'opercule 76 présente une collerette 94, visible à la figure 4, globalement annulaire qui s'étend autour de l'axe B d'injection et qui est en appui axial contre un joint élastomère (non représenté) de la buse 38, pour protéger l'extrémité libre aval de la buse 38 et notamment les canaux d'injection 42.

Un exemple de fonctionnement et un exemple de montage du dispositif 10 d'injection selon l'invention sont présentés ci-dessous.

L'opercule 76 est monté sur le disque 62 de fond du bouchon, dans une position angulaire déterminée, autour de l'axe B d'injection, l'ensemble ainsi formé est inséré axialement de bas en haut à travers le passage 60 circulaire délimité par le bouchon 54, de sorte que le disque 62 et l'opercule 76 sont solidaires du bouchon 54.

Ensuite, le bouchon 54 équipé de l'opercule 76 est monté sur l'ensemble formé par le corps 12, le capot 46 et la buse 38.

A cet effet, le bouchon 54 est présenté dans une position pivotée angulairement correspondant à sa position d'ouverture avant d'être entraîné axialement vers le haut de façon à enclencher le premier moyen de verrouillage à baïonnette du bouchon 54 sur le corps 12, et le second moyen de verrouillage à baïonnette de l'opercule 76 sur l'extrémité libre de la buse 38 d'injection.

Une fois dans sa position d'ouverture, le bouchon 54 est pivoté autour de l'axe B d'injection jusqu'à sa position de fermeture pour verrouiller simultanément le bouchon 54 sur le corps 12 et pour verrouiller l'opercule 76 sur la buse 38.

Le retrait et le déverrouillage du bouchon 54 est réalisé, à l'inverse, en entraînant le bouchon 54 depuis sa position de fermeture jusqu'à sa position d'ouverture.

Avantageusement, le dispositif 10 selon l'invention permet d'entraîner le bouchon en pivotement entre sa position d'ouverture et sa position de fermeture aussi bien dans un sens de pivotement que dans un autre sens, autour de l'axe B d'injection.

De plus, la bague 88 de blocage montée sur l'opercule 76 offre un positionnement angulaire précis de l'opercule 76 par rapport au bouchon 54, et offre également, par conséquent, un positionnement angulaire précis du bouchon 54 par rapport au capot 46, de sorte que le bouchon 54, dans sa position de fermeture, est parfaitement aligné avec le capot 46 du dispositif 10.

Concernant le fonctionnement du dispositif 10 d'injection décrit succinctement par la suite, celui-ci est similaire au fonctionnement du dispositif décrit dans le document FR-A-2815544.

L'utilisateur déverrouille le dispositif 10 d'injection en ôtant le bouchon 54 par une rotation dans un sens ou dans l'autre. Il applique l'extrémité libre de la buse 38 contre la peau du patient à traiter et, par pression d'un doigt, il enfonce le capot 46 qui coulisse le long du corps 12 jusqu'à au déclenchement du générateur de gaz 20.

Les gaz générés envahissent la chambre d'expansion 22 et, lorsque la pression est suffisante, exercent une poussée sur la colonne de liquide constituée par les deux bouchons-pistons 32, 34 et le principe actif liquide 26, le principe actif liquide 26 est alors expulsé par les canaux 42

La présente description de l'invention est donnée à titre d'exemple non limitatif.

## Revendications

1. Dispositif (10) d'injection sans aiguille comportant :
- un corps (12),
- un générateur de gaz (20),
- un système d'injection (28) qui s'étend axialement suivant un axe (B) d'injection et qui comprend au moins, d'amont en aval suivant le sens d'injection, un piston (32, 34), un réservoir (24) contenant un principe actif, une buse (38) d'injection qui délimite au moins un canal (42) d'injection,
- un capot (46) qui loge le corps (12) du dispositif (10),
- un opercule (76) amovible adapté pour protéger une extrémité aval inférieure de la buse (38) d'injection,
- un bouchon (54) qui loge l'opercule (76) et qui est monté pivotant autour de l'axe (B) d'injection entre une position de fermeture dans laquelle le bouchon (54) est verrouillé sur le dispositif (10) d'injection et une position d'ouverture dans laquelle le bouchon (54) est déverrouillé du dispositif (10) d'injection,
**caractérisé en ce que** le dispositif (10) comporte un élément de blocage (88) qui est interposé radialement entre l'opercule (76) et le bouchon (54), ledit élément de blocage (88) étant conçu pour bloquer par friction le pivotement relatif de l'opercule (76) et du bouchon (54), autour de l'axe (B) d'injection, au cours de l'entraînement en pivotement du bouchon (54).

2. Dispositif (10) d'injection sans aiguille selon la revendication 1, **caractérisé en ce que** l'élément de blocage (88) est une bague qui est fixée sur une paroi périphérique cylindrique de l'opercule (76) autour de l'axe (B) d'injection.

3. Dispositif (10) d'injection sans aiguille selon l'une quelconque des revendications précédentes, **caractérisé en ce que** l'élément de blocage (88) est réalisé en matériau déformable élastiquement.

4. Dispositif (10) d'injection sans aiguille selon l'une quelconque des revendications précédentes, **caractérisé en ce que** l'élément de blocage (88) est réalisé en élastomère.

5. Dispositif (10) d'injection sans aiguille selon l'une quelconque des revendications précédentes, **caractérisé en ce que** l'élément de blocage (88) est réalisé en surmoulage sur l'opercule (76).

6. Dispositif (10) d'injection sans aiguille selon l'une quelconque des revendications précédentes, **caractérisé en ce que** le bouchon (54) comporte une portion globalement tubulaire qui délimite un logement adapté pour loger l'opercule (76) et l'extrémité aval de la buse (38), ladite portion tubulaire étant fermée à une extrémité aval par un disque (62) de fond amovible, ledit disque (62) comportant une pluralité de pattes (64) de serrage qui s'étendent axialement depuis une face supérieure (68) du disque (62) et qui sont conçues pour être chacune en appui radial sur l'élément de blocage (88) pour pincer et bloquer l'élément de blocage (88) en pivotement.

7. Dispositif (10) d'injection sans aiguille selon la revendication 6, **caractérisé en ce que** chaque patte (64) de serrage est déformable élastiquement au moins radialement vers le centre de l'opercule (76).

8. Dispositif (10) d'injection sans aiguille selon l'une quelconque des revendications 6 ou 7, **caractérisé en ce que** chaque patte (64) de serrage présente une nervure (92) axiale, formant dent, qui est adaptée pour pénétrer radialement sensiblement dans l'élément de blocage (88) pour bloquer ledit élément de blocage (88) en pivotement par rapport au bouchon (54).

9. Dispositif (10) d'injection sans aiguille selon l'une quelconque des revendications 6 à 8, **caractérisé en ce que** chaque patte (64) de serrage est en appui radial contre une paroi interne du bouchon (54) pour contraindre lesdites pattes (64) radialement vers l'élément de blocage (88).

10. Dispositif (10) d'injection sans aiguille selon l'une quelconque des revendications précédentes, **caractérisé en ce que** l'opercule (76) présente une collerette (94) globalement annulaire qui s'étend autour de l'axe (B) d'injection et qui est adaptée pour coopérer avec l'extrémité aval de la buse (38).

11. Dispositif (10) d'injection sans aiguille selon l'une quelconque des revendications précédentes, **caractérisé en ce que** le dispositif (10) d'injection comporte un premier moyen de verrouillage du type à baïonnette adapté pour verrouiller le bouchon (54) sur le corps (12) du dispositif (10), au cours de l'entraînement du bouchon (54) entre sa position d'ouverture et sa position de fermeture.

12. Dispositif (10) d'injection sans aiguille selon l'une quelconque des revendications précédentes, **caractérisé en ce que** le dispositif (10) d'injection comporte un second moyen de verrouillage du type à baïonnette adapté pour verrouiller l'opercule (76) sur la buse (38) au cours de l'entraînement du bouchon (54) entre sa position d'ouverture et sa position de fermeture.

13. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadelloser Injektor (10), umfassend:
- einen Körper (12),
- einen Gasgenerator (20),
- ein Injektionssystem (28), das sich axial gemäß einer Injektionsachse (B) erstreckt, und das mindestens, von stromaufwärts nach stromabwärts gemäß der Injektionsrichtung, einen Kolben (32, 34), einen Behälter (24), enthaltend einen Wirkstoff, eine Injektionsdüse (38), die mindestens einen Injektionskanal (42) begrenzt, umfasst,
- eine Kappe (46), die den Körper (12) der Vorrichtung (10) aufnimmt,
- einen entfernbaren Deckel (76), der ausgelegt ist, um ein unteres nachgelagertes Ende der Injektionsdüse (38) zu schützen,
- einen Stopper (54), der den Deckel (76) aufnimmt, und der schwenkend um die Injektionsachse (B) zwischen einer Verschlussposition, in der der Stopper (54) auf der Injektionsvorrichtung (10) verriegelt ist, und einer Öffnungsposition, in der der Stopper (54) von der Injektionsvorrichtung (10) entriegelt ist, montiert ist,
**dadurch gekennzeichnet, dass** die Vorrichtung (10) ein Blockierelement (88) umfasst, das radial zwischen dem Deckel (76) und dem Stopper (54) angebracht ist, wobei das Blockierelement (88) entworfen ist, um durch Reibung das relative Schwenken des Deckels (76) und des Stoppers (54) um die Injektionsachse (B) im Laufe des Schwenkens des Stoppers (54) zu blockieren.

2. Nadelloser Injektor (10) nach Anspruch 1, **dadurch gekennzeichnet, dass** das Blockierelement ein Ring (88) ist, der auf eine zylindrische Umfangswand des Deckels (76) um die Injektionsachse (B) fixiert ist.

3. Nadelloser Injektor (10) nach einem der vorhergehenden Ansprüche, **dadurch gekennzeichnet, dass** das Blockierelement (88) aus elastisch verformbarem Material hergestellt ist.

4. Nadelloser Injektor (10) nach einem der vorhergehenden Ansprüche, **dadurch gekennzeichnet, dass** das Blockierelement (88) aus Elastomer hergestellt ist.

5. Nadelloser Injektor (10) nach einem der vorhergehenden Ansprüche, **dadurch gekennzeichnet, dass** das Blockierelement (88) durch Aufformen auf dem Deckel (76) hergestellt ist.

6. Nadelloser Injektor (10) nach einem der vorhergehenden Ansprüche, **dadurch gekennzeichnet, dass** der Stopper (54) einen im Wesentlichen rohrförmigen Abschnitt umfasst, der eine Aufnahme begrenzt, die ausgelegt ist, um den Deckel (76) und das nachgelagerte Ende der Düse (38) aufzunehmen, wobei der rohrförmige Abschnitt an einem nachgelagerten Ende durch eine Scheibe (62) mit abnehmbarem Boden geschlossen ist, wobei die Scheibe (62) eine Vielzahl von Schneidefüßen (64) umfasst, die sich axial von einer oberen Seite (68) der Scheibe (62) erstrecken, und die entworfen sind, um jeweils radial auf dem Blockierelement (88) aufzuliegen, um das Blockierelement (88) schwenkend zu greifen und zu blockieren.

7. Nadelloser Injektor (10) nach Anspruch 6, **dadurch gekennzeichnet, dass** jeder Schneidefuß (64) mindestens radial hin zum Zentrum des Deckels (76) elastisch verformbar ist.

8. Nadelloser Injektor (10) nach einem der Ansprüche 6 oder 7, **dadurch gekennzeichnet, dass** jeder Schneidefuß (64) eine axiale Rippe (92) aufweist, die einen Zahn bildet, die ausgelegt ist, um radial im Wesentlichen in das Blockierelement (88) einzudringen, um das Blockierelement (88) in der Schwenkung mit Bezug auf den Stopper (54) zu blockieren.

9. Nadelloser Injektor (10) nach einem der Ansprüche 6 bis 8, **dadurch gekennzeichnet, dass** jeder Schneidefuß (64) radial gegen eine interne Wand des Stoppers (54) aufliegt, um die Füße (64) radial hin zum Blockierelement (88) zu zwingen.

10. Nadelloser Injektor (10) nach einem der vorhergehenden Ansprüche, **dadurch gekennzeichnet, dass** der Deckel (76) einen im Allgemeinen ringförmigen Kragen (94) aufweist, der sich um die Injektionsachse (B) erstreckt, und der ausgelegt ist, um mit dem nachgelagerten Ende der Düse (38) zusammenzuarbeiten.

11. Nadelloser Injektor (10) nach einem der vorhergehenden Ansprüche, **dadurch gekennzeichnet, dass** der Injektor (10) ein erstes Verriegelungsmittel vom Typ Bajonett umfasst, das ausgelegt ist, um den Stopper (54) auf dem Körper (12) der Vorrichtung (10) im Laufe des Antriebes des Stoppers (54) zwischen seiner Öffnungsposition und seiner Verschlussposition zu verriegeln.

12. Nadelloser Injektor (10) nach einem der vorhergehenden Ansprüche, **dadurch gekennzeichnet, dass** der Injektor (10) ein zweites Verriegelungsmittel vom Typ Bajonett umfasst, das ausgelegt ist, um den Deckel (76) auf der Düse (38) im Laufe des Antriebs des Stoppers (54) zwischen seiner Öffnungsposition und seiner Verschlussposition zu verriegeln.

13. Nadelloser Injektor (10) nach einem der vorhergehenden Ansprüche, **dadurch gekennzeichnet, dass** der Wirkstoff (26), enthalten im Behälter (24), ausgewählt ist aus der Gruppe, umfassend die folgenden Wirkstoffe:
- Methotrexat,
- Adrenalin,
- Sumatriptan,
- Hydrocortison,
- Naloxon,
- Midazolam,
- Apomorphin,
- Ethylnatrexonbromid,
- Phytomenadion,
- Chlorpromazinhydrochlorid,
- Zuclopenthixolacetat,
- Danaparoid-Natrium,
- Enoxaparin-Natrium,
- Estradiolcypionat,
- Medroxyprogesteronacetat,
- Medroparin-Calcium,
- Methylprednisolonacetat,
- Heparincalcium,
- Terbulin.

## Claims

1. A needleless injection device (10) including:
- a body (12)
- a gas generator (20)
- an injection system (28) which extends axially along an injection axis (B) and which comprises at least, from upstream to downstream in the injection direction, one piston (32, 34), a reservoir (24) containing an active ingredient, an injection nozzle (38) which delimits at least one injection channel (42),
- a cover (46) which houses the body (12) of the device (10),
- a removable cap (76) adapted to protect a lower downstream end of the injection nozzle (38),
- a plug (54) which houses the cap (76) and which is pivotally mounted about the injection axis (B) between a closed position in which the plug (54) is locked on the injection device (10) and an open position in which the plug (54) is unlocked from the injection device (10), **characterized in that** the device (10) includes a blocking element (88) which is radially interposed between the cap (76) and the plug (54), said blocking element (88) being designed to frictionally block relative pivoting of the cap (76) and the plug (54) about the injection axis (B) during the driving of the plug (54) in pivoting.

2. The needleless injection device (10) according to claim 1, **characterized in that** the blocking element (88) is a ring which is fixed on a cylindrical peripheral wall of the cap (76) around the injection axis (B).

3. The needleless injection device (10) according to any one of the preceding claims, **characterized in that** the blocking element (88) is made of an elastically deformable material.

4. The needleless injection device (10) according to any one of the preceding claims, **characterized in that** the blocking element (88) is made of elastomer.

5. The needleless injection device (10) according to any one of the preceding claims, **characterized in that** the blocking element (88) is made by overmolding onto the cap (76).

6. The needleless injection device (10) according to any one of the preceding claims, **characterized in that** the plug (54) includes a generally tubular portion which delimits a housing adapted to house the cap (76) and the downstream end of the nozzle (38), said tubular portion being closed at a downstream end by a removable bottom disc (62), said disc (62) including a plurality of clamping tabs (64) which axially extend from an upper face (68) of the disc (62) and which are designed to be each radially bearing on the blocking element (88) in order to pinch and block the pivoting of the blocking element (88).

7. The needleless injection device (10) according to claim 6, **characterized in that** each clamping tab (64) is elastically deformable at least radially toward the center of the cap (76).

8. The needleless injection device (10) according to any one of claims 6 or 7, **characterized in that** each clamping tab (64) has an axial rib (92), forming a tooth, which is adapted to radially substantially penetrate in the blocking element (88) for blocking the pivoting of said blocking element (88) relative to the plug (54).

9. The needleless injection device (10) according to any one of claims 6 to 8, **characterized in that** each clamping tab (64) is radially bearing against an inner wall of the plug (54) to constrain said tabs (64) radially toward the blocking element (88).

10. The needleless injection device (10) according to any one of the preceding claims, **characterized in that** the cap (76) has a generally annular flange (94) which extends around the injection axis (B) and which is adapted to cooperate with the downstream end of the nozzle (38).

11. The needleless injection device (10) according to any one of the preceding claims, **characterized in that** the injection device (10) includes a first bayonet-type locking means adapted to lock the plug (54) on the body (12) of the device (10), during the driving of the plug (54) between its open position and its closed position.

12. The needleless injection device (10) according to any one of the preceding claims, **characterized in that** the injection device (10) includes a second bayonet-type locking means adapted to lock the cap (76) on the nozzle (38) during the driving of the plug (54) between its open position and its closed position.

13. The needleless injection device (10) according to any one of the preceding claims, **characterized in that** the active ingredient (26) contained in the reservoir (24) is selected from the group comprising of the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medroxyprogesterone acetate,
- Medroparin calcium,
- Methylprednisolone acetate
- Heparin calcium,
- Terbulin.
